**Europäisches Patentamt**

**European Patent Office**

**Office européen des brevets**

(19)

(11) Veröffentlichungsnummer : **0 052 252**
**B1**

(12) **EUROPÄISCHE PATENTSCHRIFT**

(45) Veröffentlichungstag der Patentschrift :
24.04.85

(21) Anmeldenummer : 81108666.9

(22) Anmeldetag : 22.10.81

(51) Int. Cl.⁴ : **C 12 M   3/02, C 12 M   1/12**

(54) Verfahren und Vorrichtung zur submersen Züchtung von Zellkulturen.

(30) Priorität : 18.11.80 CH 8542/80

(43) Veröffentlichungstag der Anmeldung :
26.05.82 Patentblatt 82/21

(45) Bekanntmachung des Hinweises auf die Patenterteilung : 24.04.85 Patentblatt 85/17

(84) Benannte Vertragsstaaten :
CH DE FR GB LI NL SE

(56) Entgegenhaltungen :
FR-A- 2 177 051
FR-A- 2 236 004
FR-A- 2 324 365
FR-A- 2 450 567
GB-A- 2 059 436
US-A- 3 993 581
PATENTS ABSTRACTS OF JAPAN, Band 3, Nr. 142(C-
65), 24. November 1979, Seite 159C65
CHEMICAL ABSTRACTS, Band 96, 1982, Seite 358,
Nr. 100292x, Columbus, Ohio, USA, V.V. ARKHIPOV:
"Cultivation of cells on liquid fluorocarbon substrates"

(73) Patentinhaber : Chemap AG
Hölzliwisenstrasse 5
CH-8604 Volketswil (CH)

(72) Erfinder : Karrer, Daniel, Dr.
Rinderweid 5
CH-8707 Uetikon a/See (CH)
Erfinder : Bondi, Henry S.
Bergstrasse 63
CH-8708 Männedorf (CH)

(74) Vertreter : Herrmann, Peter Johannes et al
c/o PPS Polyvalent Patent Service AG Mellingerstrasse 1
CH-5400 Baden (CH)

## Beschreibung

Die Erfindung betrifft ein Verfahren und eine Vorrichtung zur submersen Züchtung von Zellkulturen mittels indirekter Begasung über rohrförmige gasdurchlässige Membranen eines feststehenden Permeators.

Die Züchtung diploider Zellkulturen auf festen Unterlagen ist bekannt. So verwendet man als Unterlagen für die Kulturen sogenannte Rouxflaschen. Darin dient nur die über der Kultur und dem Nährsubstrat befindliche Luft zur Begasung der Kultur. Eine Weiterentwicklung bilden die Rollerflaschen, in welchen das Nährmedium langsam bewegt wird und somit auch der Gasaustausch beschleunigt wird. Auch rotierende Glasplatten oder Glasrohre, die ganz oder teilweise in ein Kulturmedium eintauchen, sind bekannt. Hierbei sind die wirksamen Oberflächen, auf denen die Gewebezellen wachsen können, bedeutend grösser.

Zur weiteren Vergrösserung der Oberfläche ist die Züchtung auf Hohlfasern beschrieben. Diese stellen halbdurchlässige rohrförmige Membranen dar.

Aus der DE-OS 2 319 120 ist ein Verfahren und eine Apparatur bekannt, bei welchen sich an den Aussenflächen eines Hohlfaserbündels Gewebezellen ansiedeln können. Die Hohlfasern bestehen aus Acetatcellulose und gut gasdurchlässigem Silan-Polycarbonat-Copolymer. Mit Sauerstoff angereicherte Nährstoffe werden so in das Innere der Hohlfasern geführt; die Stoffwechselprodukte werden aus dem Innern über die Hohlfasermembran abgeführt.

Auch die DE-OS 24 31 450 beschreibt ein Verfahren zur Kultivierung von Zellen aus Hohlfasern. Danach werden Zellen aseptisch an eine äussere oder innere Oberfläche atoxischer Hohlfasermembranen gebunden. Es wird Luft mit pulsierendem Fluss durch das Innere dieser Hohlfasermembranen geleitet. Es ist aber auch ein Messverfahren zur Steuerung der Zellzucht mittels gasdurchlässiger Membranen bekannt (Patent Abstracts of Japan, Band 3, Nr. 142 (C-65), Seite 159C65 unter 54-122790). Danach wird eine mikroporöse Membran zur Trennung der Gasphase von der Flüssigkeitsphase eingesetzt.

Ein Nachteil besteht darin, dass sich auf der entstehenden Zellschicht oft eine zweite Zellschicht bildet, die von dem Gasaustausch abgeschnitten ist und deren Zellen in Nekrose übergehen. Das gleiche trifft bei noch in Suspension befindlichen Zellen zu, die sedimentieren. Der gemeinsame Nachteil aller dieser Züchtungsmethoden mit auf einer Oberfläche fixierten Gewebezellen besteht darin, dass eine Kontrolle des physiologischen Zustandes der Kultur schlecht möglich ist.

Aufgabe der Erfindung ist es, ein submerses Verfahren zur Züchtung von Gewebezellkulturen mit indirekter Begasung über gasdurchlässige Membranen eines feststehenden Permeators zu schaffen, sowie eine Vorrichtung zur Durchführung des Verfahrens, wobei eine Kontrolle des physiologischen Zustandes der Zellen durch Messung und Regelung charakteristischer Merkmale wie pH oder $pO_2$ sowie $pCO_2$ möglich ist.

Diese Aufgabe wird nach Anspruch 1 gelöst und ist dadurch gekennzeichnet, dass die Frischgaszufuhr und die Abgasabfuhr mittels einer Flüssigkeit als Träger für $O_2/CO_2$ über den beidseitig mit gasdurchlässigen Membranen versehenen hohlen Stützkörper des Permeators erfolgt.

Der Stützkörper des Permeators besteht aus einem porösen Rohr, welches beidseitig, d. h. innen und aussen mit einer gasdurchlässigen Membran aus vorzugsweise Teflon oder Silikonpolymeren versehen ist. Aber auch andere gasdurchlässige Membranen, wenn sie keinen schädlichen Einfluss auf das Zellwachstum ausüben, können verwendet werden.

Ein im Innern des Permeators angebrachter Rührer kann sowohl von unten nach oben als auch umgekehrt fördern. Auch kann der Rührer von oben oder unten in den Permeator eingeführt sein. Als Rührwerke sind axial fördernde Propellerrührer, Schrägblattrührer und Vibrationsrührer geeignet.

Der Schlankheitsgrad eines Gefässes ist durch das Verhältnis aus Durchmesser durch Höhe des Gefässes definiert.

Für das Zellzuchtgerät hat sich ein Schlankheitsgrad von 0,3 bis 0,1, vorzugsweise 0,2 bis 0,1 als geeignet erwiesen. Auch der Permeatoreinsatz sollte einen Durchmesser von 0,5 bis 0,8 des einfachen, vorzugsweise 0,7-fachen des Behälterdurchmessers aufweisen. Die Membranstärke beträgt weniger als 0,1 mm.

Der Zellzuchtbehälter ist mit den erforderlichen Mess- und Steuergeräten für $pO_2$, $pCO_2$, pH und Temperatur versehen.

Das Verfahren und die Vorrichtung sollen anhand von Zeichnungen beschrieben werden.

Es zeigen:

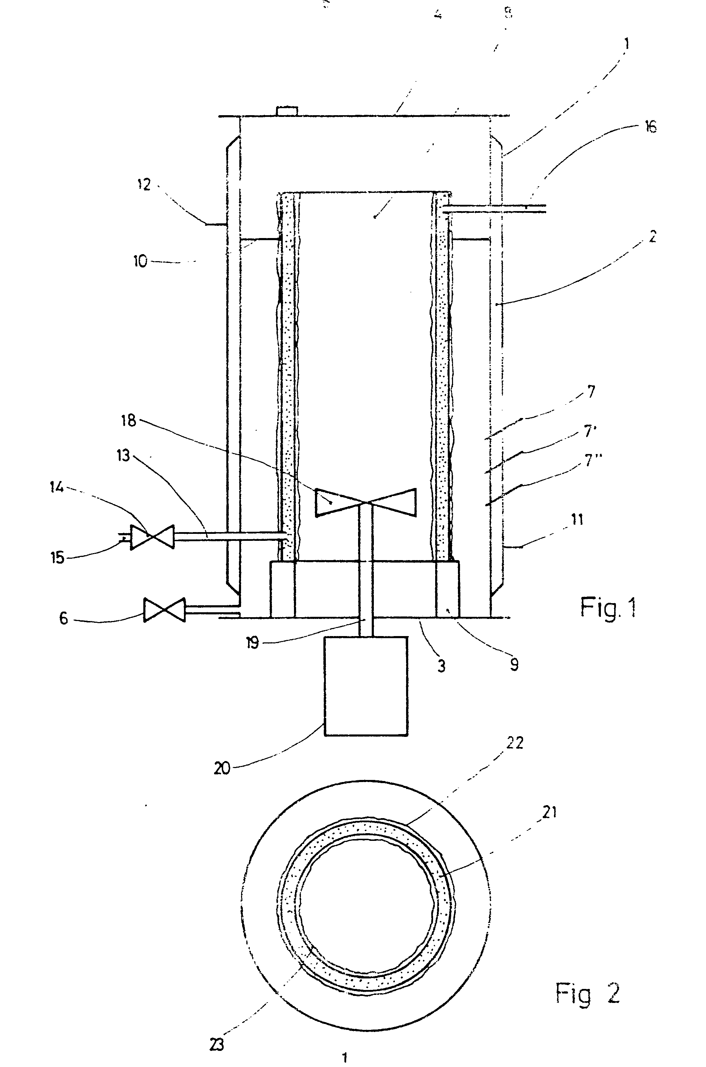

Figur 1 einen schematischen Schnitt durch das erfindungsgemässe Gewebezellzuchtgerät,

Figur 2 einen Querschnitt durch den Permeator.

Der Zellzuchtapparat besteht aus einem vertikal ausgedehnten Glas- oder Stahlbehälter 1, dessen Verhältnis Durchmesser zu Höhe 1/5 bis 1/10 betragen kann. Der Behälter 1 ist mit einem Doppelmantel 2, einem Boden 3 und einem Deckel 4 versehen. Im Deckel 4 befinden sich ein oder mehrere Sterilanschlüsse 5. Im unteren Teil des Behälters ist ein Ernteventil 6 installiert. Die Behälterwand ist ferner mit einem Stutzen 7 zur Sauerstoff- und/oder Kohlenstoffdioxidmessung, einem Stutzen 7' zur pH-Messung und einem Stutzen 7" zur Temperaturmessung vorgesehen. Im Behälter 1 ist ein Permeator 8 als zentrales Leitrohr auf Glas- oder Metallstützen 9 gelagert und in seinem oberen Teil über eine oder mehrere Verstrebungen 10 befestigt. Der Permeator 8 besteht in seinen wesentlichen Teilen aus einem hohlen beispielsweise porösen Stützkörper 21, aus einer äusseren gasdurchlässigen Membran 22 und einer inneren gasdurchlässigen Membran 23. Der Doppelmantel ist mit einem Eingangsstutzen 11 und einem Ausgangsstutzen 12 für das Wärmeaustauschmittel versehen. Der Permeator 8 ist über einen Leitungsteil 13 und ein Ventil 14 mit einer Gassammelleitung 15 verbunden. Die Leitung 15 für Frischgas zusammen mit der Flüssigkeit als Träger wird von einer nicht gezeigten Gasdosierungsvorrichtung gespeist. Eine Leitung 16 für das Abgas zusammen mit der Flüssigkeit als Träger führt ins Freie. Ein Rührwerk 18 ist im unteren Teil des Permeators befestigt und über eine Antriebswelle 19 mit einem Elektromotor 20 verbunden.

Im Betrieb ist der Behälter 1 des Gewebezellzuchtgerätes mit vorher sterilisiertem Nährsubstrat und Gewebezellen, insbesondere mit diploiden Humanzellen befüllt. Der Rührer 18 hält die Zellen in Suspension. Gleichzeitig strömt Gas, welches aus maximal 5 Vol.% $CO_2$, bis 20 Vol.% $O_2$ und bis 50 Vol.% $N_2$ besteht, der Rest weist die Zusammensetzung von Luft auf, aus einer nicht gezeigten Mischbatterie zusammen mit einer Flüssigkeit als Träger in die Sammelleitung 15, das Ventil 14 und die Leitung 13 in die Hohlräume des porösen Stützkörpers 21. Sowohl durch die äussere Membran 22 als auch durch die innere Membran 23 vermag der Sauerstoff in die Nährlösung zu diffundieren und gleichzeitig $CO_2$ aus der Nährlösung in das Innere des hohlen Stützkörpers zurückdiffundieren. Die mit $CO_2$ auf diese Weise angereicherte Trägerflüssigkeit verlässt das Innere zwischen beiden Membranen über die Leitung 16.

Es findet ein reger Gasaustausch statt, ohne dass Luftbläschen in das Nährsubstrat treten und als solche mit den Gewebezellen in Berührung kommen. Auch wird auf diese Weise jede Schaumbildung vermieden.

Als flüssige Träger kommen beispielsweise fluorierte Kohlenwasserstoffe oder komplette Nährmedien infrage.

Das erfindungsgemässe Verfahren kann neben rein submerser Züchtung unter Verwendung von Microcarriern in gleicher Weise durchgeführt werden.

Die erfindungsgemässe Vorrichtung weist zahlreiche günstige Eigenschaften auf :

— grosse Stoffaustauschfläche
— definiertes Strömungsmuster im Reaktor
— gute Homogenität der Suspension
— geringe Scherkräfte durch Verwendung eines langsam laufenden Axial-Rührers (Sedimentation der Carrier muss verhindert werden)
— sehr einfaches und leicht zu kalkulierendes scaling-up
— keine Schaumprobleme, daher keine Zellanreicherung im Schaum
— kleine Serumkonzentration, da Scherkräfte klein
— Kontrolle von $pCO_2$ und $pO_2$ möglich
— Verwendung von reinem Sauerstoff zur Begasung möglich
— geringer Mediumverbrauch durch Kombination von Permeator und Reaktor
— bei hohem $O_2$-Bedarf konzentrische Zentralrohre möglich zur Vergrösserung der Oberflächen
— einfache Reinigung
— bessere Ausnutzung des Fermentervolumens
— in situ sterilisierbar.

## Patentansprüche

1. Verfahren zur submersen Züchtung von Zellkulturen mittels indirekter Begasung über rohrförmige gasdurchlässige Membranen eines feststehenden Permeators (8), dadurch gekennzeichnet, dass die Frischgaszufuhr und die Abgasabfuhr mittels einer Flüssigkeit als Träger für $O_2$/$CO_2$ über den beidseitig mit gasdurchlässigen Membranen (22, 23) versehenen hohlen Stützkörper (21) des Permeators (8) erfolgt.

2. Verfahren nach Anspruch 1, dadurch gekennzeichnet, dass als Trägerflüssigkeit ein fluorierter Kohlenwasserstoff verwendet wird.

3. Verfahren nach Anspruch 2, dadurch gekennzeichnet, dass als Trägerflüssigkeit das Nährmedium selbst verwendet wird.

4. Vorrichtung zur Durchführung des Verfahrens nach Anspruch 1, dadurch gekennzeichnet, dass der rohrförmige Permeator (8) ein senkrechtes Zentralrohr im Behälter (1) des Zellzuchtgerätes bildet.

5. Vorrichtung nach Anspruch 4, dadurch gekennzeichnet, dass der Stützkörper (21) des Permeators (8) an seiner Aussenseite mit einer gasdurchlässigen Membran (23) und an seiner Innenseite mit einer gasdurchlässigen Membran (22) versehen ist.

6. Vorrichtung nach den Ansprüchen 4 und 5, dadurch gekennzeichnet, dass im Innern des Permeators (8) ein Rührer (18) angebracht ist.

7. Vorrichtung nach den Ansprüchen 4 bis 6, dadurch gekennzeichnet, dass der hohle Stützkörper (21) aus einem porösen Zylinder besteht.

8. Vorrichtung nach den Ansprüchen 4 bis 6, dadurch gekennzeichnet, dass der hohle Stützkörper (21) aus einem perforierten Rohr besteht.

9. Vorrichtung nach Anspruch 4, dadurch gekennzeichnet, dass der Behälter (1) des Zellzuchtgeräts einen Schlankheitsgrad von 0,3 bis 0,1 aufweist.

10. Vorrichtung nach Anspruch 4, dadurch gekennzeichnet, dass der Permeator (8) das 0,5 bis 0,8-fache des Durchmessers des Behälters (1) aufweist.

11. Vorrichtung nach den Ansprüchen 4 bis 10, dadurch gekennzeichnet, dass die Dicke der gasdurchlässigen Membranen weniger als 0,1 mm beträgt.

12. Vorrichtung nach den Ansprüchen 4 bis 11, dadurch gekennzeichnet, dass der Permeator (8) ein zentrales Leitrohr bildet, dessen obere und untere Kreisringflächen gasdicht verschlossen sind.

## Claims

1. Method for the submersed growth of cell cultures by means of indirect aeration across tubular gas-permeable membranes of a stationary permeator (8), characterized in that fresh gas supply and waste gas removal take place by means of a liquid as carrier of $O_2/CO_2$ across the hollow support body (21) of the permeator (8) provided on both sides with gas-permeable membranes (22, 23).

2. Method according to claim 1, wherein a fluorinated hydrocarbon is used as the carrier liquid.

3. Method according to claim 2, wherein the nutrient medium alone is used as the carrier liquid.

4. Apparatus for carrying out the method according to claim 1, wherein the tubular permeator (8), is a perpendicular central tube inside the container (1) of the cell culture device.

5. Apparatus according to claim 4, wherein the hollow support body (21) of the permeator (8) is provided with a gas-permeable membrane (23) on its outer side and a gas-permeable membrane (22) on its inner side.

6. Apparatus according to the claims 4 and 5, wherein a stirrer (18) is fixed inside the permeator (8).

7. Apparatus according to the claims 4 to 6, wherein the hollow support body (21) is a porous cylinder.

8. Apparatus according to the claims 4 to 6, wherein the hollow support body (21) is a perforated tube.

9. Apparatus according to claim 4, wherein the container (1) of the cell culture device has a fineness ratio of 0,3 to 0,1.

10. Apparatus according to claim 4, wherein the permeator (8) has a diameter of from 0,5 to 0,8 times the diameter of the container (1).

11. Apparatus according to the claims 4 to 10, wherein the thickness of said gas-permeable membranes amounts to less than about 0,1 mm.

12. Apparatus according to the claims 4 to 11, wherein the permeator (8) is a central tube which is closed gas-tight at the top and the bottom of its ring shaped surface.

## Revendications

1. Procédé de culture en immersion de cellules mettant en œuvre une alimentation gazeuse indirecte par des membranes tubulaires perméables aux gaz d'un perméateur (8) stationnaire, caractérisé en ce que l'alimentation en gaz frais et l'évacuation du gaz résiduaire sont effectués au moyen d'un fluide servant de porteur pour $O_2/CO_2$, par le support creux (21) du perméateur (8), pourvu de part et d'autre de membranes (22, 23) perméables aux gaz.

2. Procédé selon la revendication 2, caractérisé en ce que l'on utilise un hydrocarbure fluoré en tant que fluide porteur.

3. Procédé selon la revendication 2, caractérisé en ce que l'on utilise le milieu nutritif lui-même en tant que fluide porteur.

4. Dispositif pour la mise en œuvre du procédé selon la revendication 1, caractérisé en ce que le perméateur (8) tubulaire forme un tube central vertical dans le récipient (1) du dispositif de culture cellulaire.

5. Dispositif selon la revendication 4, caractérisé en ce que le support creux (21) du perméateur (8) est pourvu à sa face extérieure d'une membrane (23) perméable aux gaz et à sa face intérieure d'une membrane (22) perméable aux gaz.

6. Dispositif selon les revendications 4 et 5, caractérisé en ce qu'un agitateur (18) est monté à l'intérieur du perméateur (8).

7. Dispositif selon les revendications 4 à 6, caractérisé en ce que le support creux (21) est constitué par un cylindre poreux.

8. Dispositif selon les revendications 4 à 6, caractérisé en ce que le support creux (21) est constitué par un tube perforé.

9. Dispositif selon la revendication 4, caractérisé en ce que le récipient (1) du dispositif de culture cellulaire présente un degré d'allongement de 0,3 à 0,1.

10. Dispositif selon la revendication 4, caractérisé en ce que le diamètre du perméateur (8) présente 0,5 à 0,8 fois le diamètre du récipient (1).

11. Dispositif selon les revendications 4 à 10, caractérisé en ce que l'épaisseur des membranes perméables aux gaz est inférieure à 0,1 mm.

12. Dispositif selon les revendications 4 à 11, caractérisé en ce que le perméateur (8) définit un conduit central dont les surfaces annulaires supérieure et inférieure sont fermées d'une manière étanche aux gaz.

Fig. 1

Fig 2